# EUROPEAN PATENT SPECIFICATION

(11) **EP 0 781 569 B1**
(45) Date of publication and mention of the grant of the patent: **20.08.2003**
(21) Application number: 96660102.3
(22) Date of filing: 18.12.1996
(51) Int. Cl.: A61M 16/18

(54) **Arrangement for preventing overfill of an anaesthetic liquid container**
Vorrichtung zur Vermeidung der Überfüllung eines Narkoseflüssigkeitsbehälters
Dispositif de limitation de remplissage d'un récipient contenant un liquide anesthésique

(30) Priority: 29.12.1995 FI 956354; 18.04.1996 FI 961698
(43) Date of publication of application: 02.07.1997
(73) Proprietor: Instrumentarium Corporation, 00510 Helsinki (FI)
(72) Inventor: Heinonen, Erkki, 00750 Helsinki (FI); Kankkunen, Jukka, 01730 Vantaa (FI)
(74) Representative: Valkeiskangas, Tapio Lassi Paavali

(56) References cited:
- EP-A- 0 664 138
- DE-A- 4 106 758
- DE-C- 3 544 302
- US-A- 5 478 506

## Description

The invention relates to an arrangement for preventing overfill of an anaesthetic liquid container, comprising means for guiding anaesthetic liquid to the anaesthetic liquid container for vaporization and for removing a volume of gas equivalent to the filling of anaesthetic liquid from the anaesthetic liquid container.

In order for anaesthetic vaporizers functioning on the bypass-saturation principle to operate correctly, the amount of liquid in the liquid container should not exceed an allowable level. If the level should be exceeded, the liquid might block the flow conduit which passes through the liquid container and carries the gas flow to be supplied to the patient. The gas flow can, however, pressurize the liquid container, forcing thus extra liquid out, which results in serious over-administration. For the above reasons, to limit the amount of liquid, vaporizers are provided with a device which prevents them from being filled over the maximum allowable amount of liquid.

The filling of the liquid container of an anaesthetic vaporizer is based on the exchange of volumes in the vaporizer and the supply container of the liquid. When liquid flows into the vaporizer, an equivalent volume of gas flows out of it. Correspondingly, when liquid flows out of the supply container, an equivalent volume of gas flows into the it. The filling of the vaporizer stops if either or both of the replacement gas flows is/are exhausted.

There are three different types of systems for filling vaporizers: funnel, filler and tube.

In a funnel-type system the anaesthetic liquid is poured from the supply container, i.e. a bottle, to the funnel openly through the external atmosphere. The filling of the container stops, when the liquid surface closes the opening of the funnel that faces the vaporizer, and the gas flow is prevented from flowing out of the vaporizer. Such a system is cescribed, for example, in British Patent No. 1 224 478.

A filler-type system is used to connect the vaporizer and the bottle to each other. In this case, the filling system is closed, and anaesthetic liquid is not allowed to vaporize to the external atmosphere. The filler comprises two tubes: one carries liquid from the bottle to the vaporizer, and the other carries gas from the vaporizer to the bottle. The filler is also standardized, e.g. prEN 1280. The filling of the container stops when the vaporizer is filled to such an extent that the liquid surface reaches the gas conduit.

The tube-type system is also a closed system. In this system, the filling head of the bottle is placed in a filling device provided in the vaporizer. A tight conduit is thus formed between the bottle and the vaporizer, and the valves in both the bottle and the vaporizer are opened. Such a system is disclosed, for example, in PCT publications WO 92/12753 and WO 95/08361. The filling stops when the vaporizer is filled such that the liquid surface reaches the upper edge of the tube.

The solutions described above have the common feature that they are all positioned on a side of the vaporizer. The vaporizer can thus be overfilled if it is tilted in such a way that the edge facing the filling device rises relative to the liquid container. If the filling device falls below the liquid container as a result of tilting, the filling will stop too soon, which does not represent a safety risk in the operation of the device.

Finnish Patent No. 94097 provides a solution for the problem described above. According to this solution, the liquid conduit is provided with an inclined portion in which a ball is positioned. When the vaporizer is tilted such that the filling device rises above the liquid container, the inclination direction of the inclined conduit changes, and the ball in the conduit rolls to a sealing surface at the other end of the conduit, closing thus the liquid flow conduit. This solution is particularly suitable for filler-type devices, in which the liquid and gas flow in opposite directions in separate conduits. It is not suitable for arrangements in which the liquid and gas flow in the same conduit.

Other typical known solutions are described in DE 35 44 302 C and DE 41 06 758 A.

The object of the present invention is to provide an arrangement by which the drawbacks of the prior art can be obviated. This is achieved with an arrangement of the invention as defined in claim 1, which is characterized in that an intermediate container which is formed from a curved tubular portion being substantially in a horizontal plane in relation to the normal operative position of the anaesthetic liquid container is provided in connection with the anaesthetic liquid container, said intermediate container forming a flow path between the supply container and the interior of the anaesthetic liquid container said intermediate container being arranged to form a common conduit for the anaesthetic liquid and the gas removed from the anaesthetic liquid container, and said intermediate container defining a liquid threshold and a gas threshold of the filling port and the anaesthetic liquid container and combining the liquid threshold and the gas threshold of the filling port and the anaesthetic liquid container in such a way that the anaesthetic liquid is allowed to flow into the anaesthetic liquid container only when the liquid flow outlet level is below the gas flow outlet level.

It is an advantage of the invention that it allows overfill to be prevented in all the above-mentioned different types of filling systems. In the case of a filler-type system, the vaporizer-emptying property is lost, but during the filling the invention works well even in this system. Another advantage of the invention is its simplicity, whereby the start-up and operating costs will be low. On account of the simplicity, the arrangement is also reliable.

In the following, the invention will be described in greater detail by means of the preferred embodiments illustrated in the accompanying drawings, in which
Figure 1 is a schematic side view of an arrangement of the invention during filling in normal position,
Figure 2 is a view of the arrangement of Figure 1 in the longitudinal direction of the container,
Figure 3 is a schematic side view of the arrangement of Figures 1 and 2 during filling in which the container is tilted backwards,
Figure 4 is a view of the situation of Figure 3 in the longitudinal direction of the container,
Figures 5 and 6 are schematic views of the correlations associated with the closing angle,
Figures 7 and 8 are schematic views of a second embodiment of the invention from different directions,
Figures 9 and 10 are schematic views of a third embodiment of the invention from different directions,
Figures 11 to 14 show examples of different possible shapes of an intermediate container,
Figures 15 to 16 are schematic views of a fourth embodiment of the invention from different directions, and
Figures 17 to 20 show different variations of the embodiment of Figures 15 and 16.

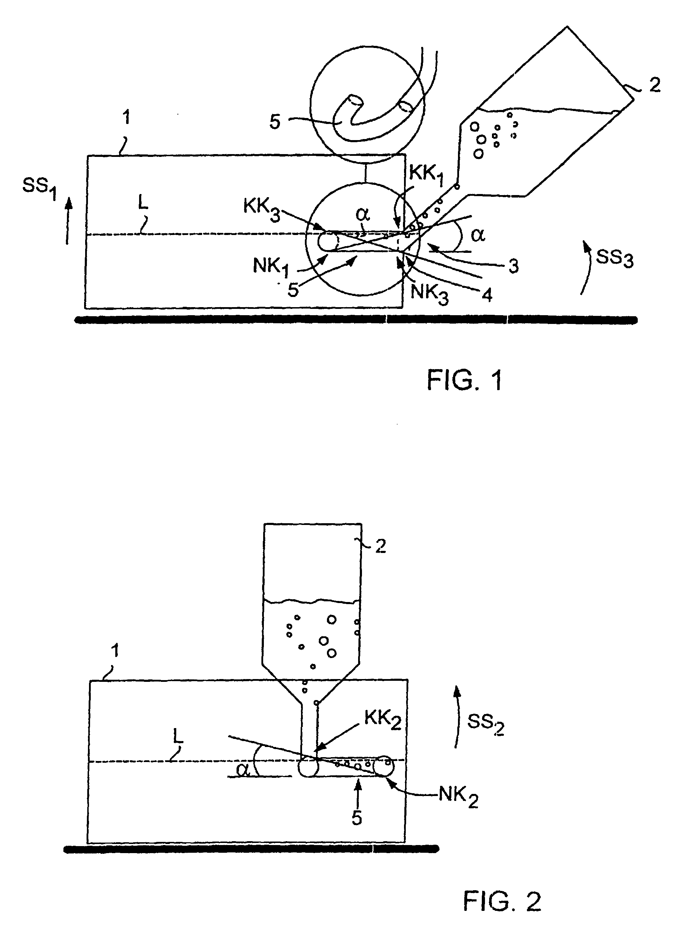

Figures 1 to 4 illustrate a preferred embodiment of the invention. Reference numeral 1 indicates an anaesthetic liquid container into which anaesthetic liquid is supplied for vaporization. The anaesthetic liquid is supplied into the anaesthetic liquid container from a supply container 2, such as a bottle. The arrangement further comprises means 3 for guiding the anaesthetic liquid into the anaesthetic liquid container 1 for vaporization and for removing an amount of gas equivalent to the filling of liquid from the anaesthetic liquid container 1 and for conducting the replacement gas into the supply container 2. The means 3 comprise, for example, a conduit through which the anaesthetic liquid and the volume of gas equivalent to the volume of liquid can flow into and out of the container, and necessary connector means for fixing the supply container 2 tightly to the filling port 4 of the anaesthetic liquid container.

The above-mentioned features represent fully conventional technology to one skilled in the art, wherefore they will not be described more closely herein. With respect to these features, reference is made, for example, to the above-mentioned publications representing the background art.

The essential feature of the invention is that the anaesthetic liquid container 1 is provided with an intermediate container 5 which is formed from a curved tubular member and which is substantially horizontal in relation to the normal operative position of the anaesthetic liquid container 1. The intermediate container 5 is arranged to provide a common conduit for the anaesthetic liquid and the gas removed from the anaesthetic liquid container 1. In the embodiment of Figures 1 to 4, the intermediate container is mounted between the filling port and the inner space of the anaesthetic liquid container. However, this is not the only possibility, but the intermediate container may also belong, for example, to the supply container. The intermediate container 5 is arranged to combine the outlet level for the liquid flow, i.e. the liquid threshold NK, and the outlet level of the gas flow, i.e. the gas threshold KK, in the filling port 4 and the anaesthetic liquid container 1 in such a way that the anaesthetic liquid is allowed to flow to the anaesthetic liquid container 1 only when the liquid flow outlet level NK is below the gas flow outlet level KK. In Figures 1 and 2, the subindexes 1 to 3 with the outlet levels NK and KK for the liquid and gas flows refer to the similarly indexed protection directions SS, i.e. the directions of the inclinations to be protected.

The solution is thus based on an intermediate container 5 formed from a curved tubular member. In the embodiment illustrated in Figures 1 to 4, the intermediate container 5 is positioned between the filling port 4 and the liquid container 1. The intermediate container 5 contains a common flow conduit for the liquid and gas flow. Thus the liquid flow outlet level NK and the gas flow outlet level KK are combined in a certain way, i.e. the liquid is allowed to flow from the bottle 2 to the liquid container 1 of the vaporizer only when the liquid flow outlet level NK is below the gas flow outlet level KK. Otherwise the intermediate container 5 is filled with liquid until the liquid surface reaches the gas flow outlet level KK, whereby the flow of replacement gas to the bottle 2 is cut off and the filling stops. The intermediate container 5 is mounted in the vaporizer in such a manner that the liquid flow outlet level, i.e. the liquid threshold NK, is below the gas flow outlet level, i.e. the gas threshold KK, in the allowable filling position, and rises above it if the vaporizer is turned to a position in which filling is not allowable.

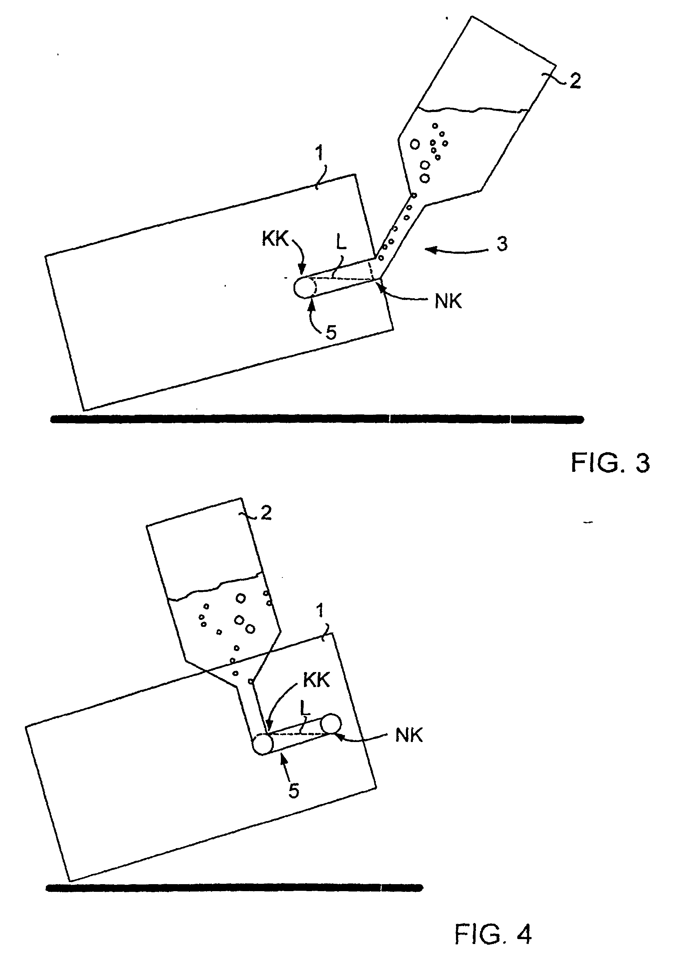

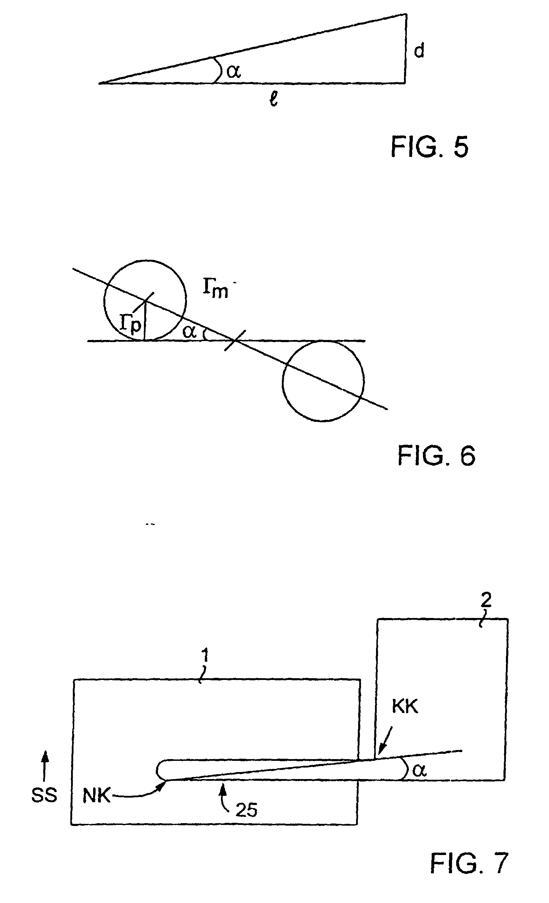

In the example shown in the figures, the intermediate container 5 is made from a substantially U-shaped tube which is provided as an extension of the filling tube and which is preferably horizontal in relation to the normal operative position of the anaesthetic liquid container. The volume of the U-shaped tube serves as the intermediate container 5. The shape of the tube can be clearly seen from Figure 1, in which the circled portion is also shown as a schematic perspective view. The liquid flow outlet level, i.e. the liquid threshold NK, is the highest point of the bottom of the flow conduit formed by the intermediate container 5. The gas flow outlet level, i.e. the gas threshold KK, in turn, is the lowest point of the top of the above-mentioned flow conduit. In the U-shaped tube according to the example shown in the figure, the liquid threshold NK is formed at the opening of the tube facing the vaporizer, whereas the gas threshold is formed in the upper part of the tube, at the locations indicated in the figures. When the vaporizer is tilted in such a way that the filling device rises above the liquid container 1, the liquid threshold NK rises above the gas threshold KK, and the filling stops. When the liquid flows from the supply container 2 towards the vaporizer, a liquid seal will be formed in the bend of the U-shaped tube which forms the intermediate container 5. This liquid seal will prevent replacement gas from flowing to the supply container 2, whereby filling is prevented when the vaporizer is tilted. Such a situation is shown as a schematic view in Figures 3 and 4. The level of the liquid surface in which the filling stops is indicated in the figures by a broken line L. In addition, Figures 1 and 2 show the closing angle α whose significance is illustrated in principle in Figures 5 and 6.

In Figure 5, the term 1 represents the length of the straight portion of the tube. In the bend of the tube, length may herein also refer to the length of its projection. The term d represents the diameter of the tube.

It can be seen from Figure 5, that 1 = dcotα. For example, if d = 7 mm and α = 10°, then 1 = 40 mm. In reality, the closing angle will be smaller because of the surface tension and viscosity of the liquid. If filling is to be prevented even in the inverted position, the flow conduit must comprise a portion whose projection in vertical direction is greater than the diameter of the tube and which in the normal filling position extends downwards.

Figure 6 illustrates the correlation between the diameter of the tube and the radius of curvature rₘ of the tube bend. It appears from Figure 6 that the correlation between the tube diameter rₚ and the radius of curvature rₘ of the tube bend is determined by the formula sinα = rₚ/rₘ.

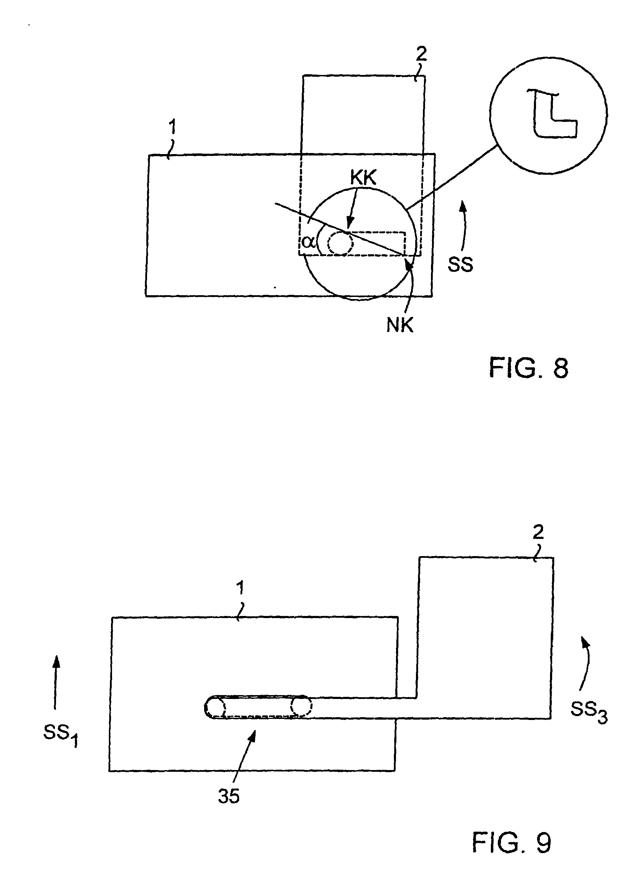

Figures 7 and 8 illustrate a second preferred embodiment of the arrangement of the invention. In this embodiment, the intermediate container 25 consists of a tubular portion which forms a bend of 90°, the bend being on a substantially horizontal plane in relation to the normal operative position of the anaesthetic liquid container. The liquid flow outlet level NK, the gas flow outlet level KK and the closing angle α are also indicated in Figures 7 and 8 in the same way as in Figures 1 to 6.

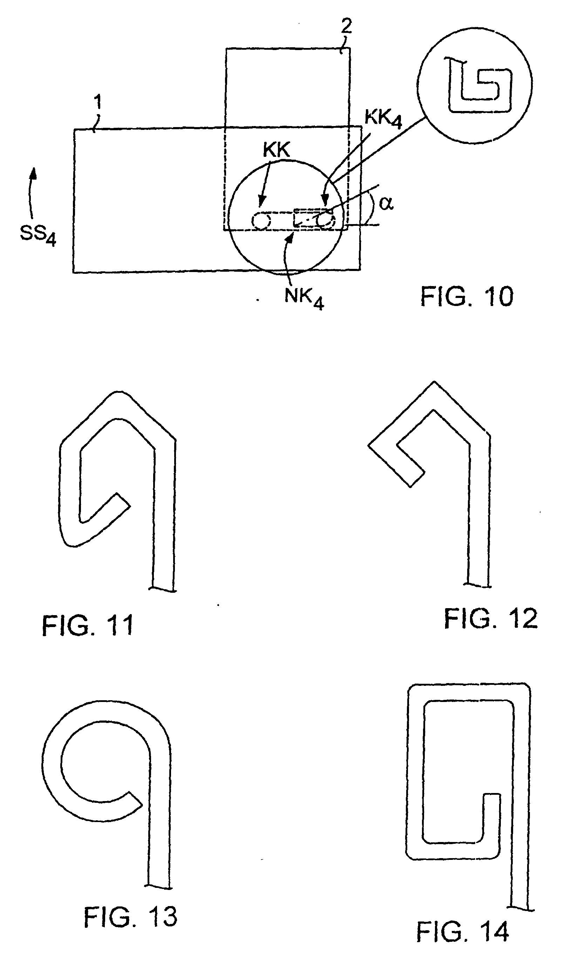

Figures 9 and 10 illustrate a third preferred embodiment of the arrangement of the invention. In this embodiment, the intermediate container 35 consists of a substantially horizontal tubular portion which forms a bend of 270°. The shape of the tubular portion is shown in Figure 10 in the same way as in Figures 7 and 8. Some of the liquid flow outlet levels NK, gas flow outlet levels KK and closing angles α are indicated in Figures 9 and 10 on the same grounds as in the preceding examples. In Figure 10, subindex 4 refers to a corresponding protection direction SS₄. As regards protection directions SS₁ and SS₃, the outlet levels NK and KK and the closing angles α are the same as in the embodiment of Figures 1 and 2, where the tubular portion forms a bend of 180°. In the protection direction, the liquid would have to flow uphill and the gas downhill.

It should thus be noted that the intermediate container can be formed in many different ways. In its simplest, the tubular portion forming the intermediate container can be, for example, a tube as shown in Figures 1 and 7, which should however be carefully positioned. The curved tubular portion, in turn, may form a bend of e.g. 90°, 180°, 270°, 360° or even greater. The curved shape can be provided in different ways, even by means of a tube that turns stepwise. The term 'curved' should be understood as a principle, the implementation of which may vary as shown below in Figures 11 to 14 and 15 to 20, for example. The tubular portion can be bent so as to form a spiral. If the bending angle is 270° or greater, a liquid seal is formed irrespective of the inclination angle and the position of the intermediate container. With smaller bending angles, a liquid seal is not formed in all inclination directions with the same inclination angles, but the intermediate container must then be placed in a correct position in the container to obtain a good result.

As stated above, the shape of the tubular portion forming the intermediate container may vary. Figures 11 to 14 show additional examples of various shapes of intermediate containers. The desired liquid level is determined by the positioning of the intermediate container in the vertical direction of the liquid container. When the container is filled above the liquid threshold, the liquid surface will form the liquid threshold. When the liquid surface rises to the level of the gas threshold, the filling stops. The stopping of the filling also depends on the viscosity and surface tension of the liquid.

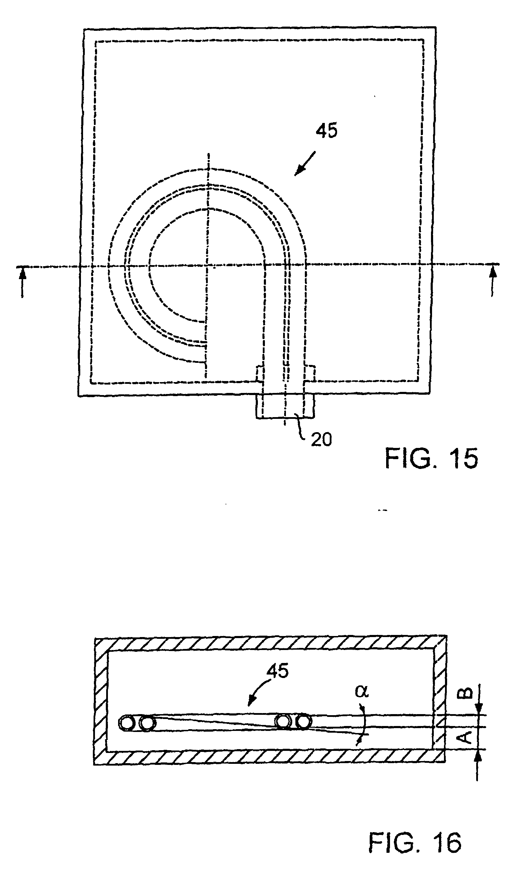

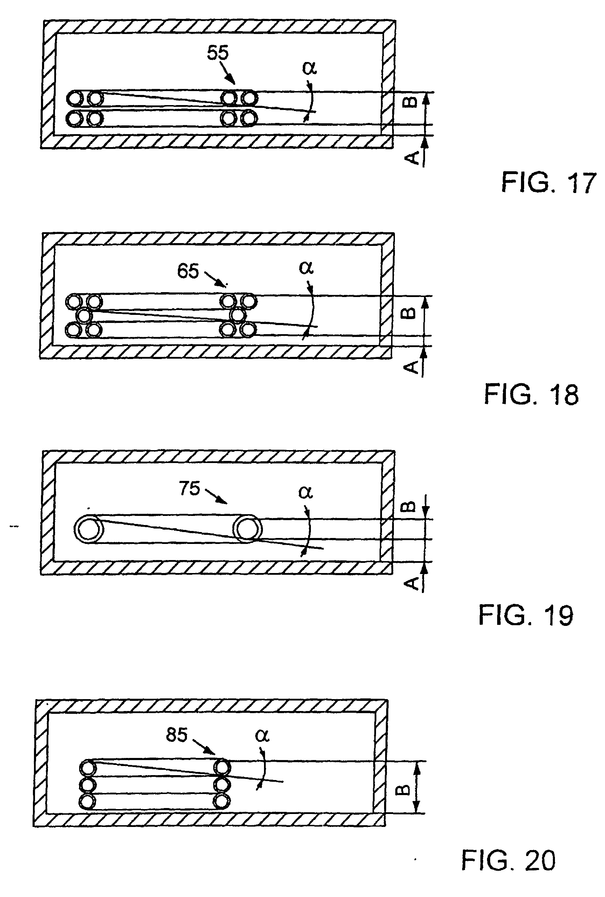

Figures 15 and 16 are schematic views of a fourth embodiment of the invention. Figures 17 to 20, in turn, illustrate variations of the embodiment of Figures 15 and 16.

In the embodiments described above, the intermediate containers are made from one tube. However, this is not the only possibility. In the embodiment of Figures 15 and 16, the intermediate container 45 is formed from two tubes which are substantially horizontal in relation to the normal operative position of the anaesthetic liquid container and which are mounted on the same plane, parallel to one another. The cross-section shape of the tubes can be selected fully freely: it may be circular, square, etc. The embodiment of Figures 15 and 16 has the advantage that the maximum surface level is can be accurately controlled, and the container is filled close to the maximum level at an even rate. In Figure 16, letter A indicates the area in which filling takes place at a constant rate, and letter B the area in which filling takes place at a rate which decelerates according to the degree of fullness. It should be noted that a small inclination angle α is achieved with this solution. There may also be more than two parallel tubes. A construction with a plurality of tubes can be preferably implemented by means of a portion forming a common inlet space 20, as shown in Figure 15.

Figures 17 and 18 show different variations of the embodiment of Figures 15 and 16. In the solutions of Figures 17 and 18, the tubes that form the intermediate container 55, 65 are on different planes. In these solutions, too, the cross-section shapes of the tubes can be fully freely selected. The solutions of Figures 17 and 18 operate in the same way as the solution of Figures 15 and 16. The difference is that the area B of decelerating filling is larger than in the solution of Figures 15 and 16. When the liquid surface rises to the lowest tube, the liquid flow starts to slow down, and it stops when the liquid surface reaches the upper surface of the inner opening of the uppermost tube.

Figure 19 shows a variation of the solutions of Figures 15-16 and 17-18. In this solution, the intermediate container 75 is made from one tube in such a way that the area of the tube is equal to the combined area of the tubes in the solution of Figure 17, for example. A greater inclination angle is achieved with the solution of Figure 19 than, for instance, with the solution of Figure 17.

Figure 20 illustrates a solution in which the tubes forming the intermediate container 85 are mounted on different planes on top of one another. In this solution, the filling rate decelerates as the degree of fullness grows, i.e. when one tube is blocked, the filling rate decelerates accordingly. In this solution, too, the cross-section shapes of the tubes can be selected freely. In the examples of the figures, the tubes are mounted in succession, parallelly and/or on top of one another. The tubes can be mounted on the same plane or on different planes, or both on the same and on different planes.

The examples described above are in no way intended to limit the invention, but the invention may be modified fully freely within the scope of the appended claims. It will therefore be clear that the arrangement of the invention or its details need not be precisely as shown in the figures, but other solutions are also possible. According to the figures, the intermediate container is made from a tube with a circular cross-section. As has already been stated in connection with some examples, it is not necessary to use a tube with a circular cross-section, but the intermediate container may also be formed from a tube whose cross-section is square, oval, triangular, etc.

## Claims

1. Arrangement with an anaesthetic liquid container of a vaporizer, comprising means for guiding anaesthetic liquid to the anaesthetic liquid container (1) for vaporization and for removing a volume of gas equivalent to the filling of anaesthetic liquid from the anaesthetic liquid container (1), the arrangement being arranged for filling the anaesthetic liquid container (1) of a vaporizer with anaesthetic liquid to a desired level from a supply container (2), said anaesthetic liquid container having a normal operative orientation with respect to a horizontal plane, said arrangement limiting the filling of the anaesthetic liquid container when the anaesthetic liquid container is tilted with respect to the horizontal plane, **characterized in that** an intermediate container (5, 25, 35, 45, 55, 65, 75, 85) which is formed from a curved tubular portion being substantially in a horizontal plane in relation to the normal operative position of the anaesthetic liquid container (1) is provided in connection with the anaesthetic liquid container (1), said intermediate container forming a flow path between the supply container (2) and the interior of the anaesthetic liquid container (1) said intermediate container (5, 25, 35, 45, 55, 65, 75, 85) being arranged to form a common conduit for the anaesthetic liquid and the gas removed from the anaesthetic liquid container, and said intermediate container (5, 25, 35, 45, 55, 65, 75, 85) defining a liquid threshold (NK) and a gas threshold (KK) of the filling port (4) and the anaesthetic liquid container (1) and combining the liquid threshold (NK) and the gas threshold (KK) of the filling port (4) and the anaesthetic liquid container (1) such that the anaesthetic liquid is allowed to flow into the anaesthetic liquid container (1) only when the liquid threshold (NK) is below the gas threshold (KK).

2. An arrangement according to claim 1, **characterized in that** the curved tubular portion is bent by at least 90° on a substantially horizontal plane in relation to the normal operative position of the anaesthetic liquid container (1).

3. An arrangement according to claim 1, **characterized in that** the intermediate container (5) is a substantially U-shaped tubular portion.

4. An arrangement according to claim 3, **characterized in that** the branches of the U-shaped tubular portion are mounted in a substantially horizontal position in relation to the normal operative position of the anaesthetic liquid container (1).

5. An arrangement according to claim 3, **characterized in that** the highest point of the bottom portion of the intermediate container (5) forms the liquid threshold (NK), and the lowest point of the upper portion forms the gas threshold level (KK).

6. An arrangement according to claim 1, **characterized in that** the curved tubular portion is shaped as a spiral.

7. An arrangement according to any one of preceding claims 1 to 6, **characterized in that** the tubular portion which forms the intermediate container (5, 25, 35, 75) is made from one tube.

8. An arrangement according to any one of preceding claims 1 to 6, **characterized in that** the tubular portion which forms the intermediate container (45, 55, 65, 85) is made from two or more tubes.

9. An arrangement according to claim 8, **characterized in that** the tubes are mounted on the same plane.

10. An arrangement according to claim 8, **characterized in that** the tubes are mounted on different planes.

11. An arrangement according to claim 8, 9 or 10, **characterized in that** the tubes are mounted in succession, parallelly and/or on top of one another.

12. An arrangement according to claim 11, **characterized in that** the tubes are mounted both on the same plane and on different planes.

13. An arrangement according to any one of the preceding claims, **characterized in that** the intermediate container (5, 25, 35, 45, 55, 65, 75, 85) is mounted between the filling port (4) and the inner space of the anaesthetic liquid container (1).

14. An arrangement according to claim 1, **characterized in that** the intermediate container comprises a portion whose projection in the vertical direction is greater than the diameter of the tube.

## Patentansprüche

1. Anordnung mit einem Narkosemittelflüssigkeitsbehälter eines Verdampfers, umfassend Mittel, um Narkosemittelflüssigkeit zur Verdampfung zu dem Narkosemittelflüssigkeitsbehälter (1) zu führen und um ein Gasvolumen zu entfernen, welches der Füllung an Narkosemittelflüssigkeit von dem Narkosemittelflüssigkeitsbehälter (1) äquivalent ist, welche Anordnung zur Füllung des Narkosemittelflüssigkeitsbehälters (1) eines Verdampfers mit Narkosemittelflüssigkeit von einem Vorratsbehälter (2) bis zu einem gewünschten Level eingerichtet ist, welcher Narkosemittelflüssigkeitsbehälter bezüglich einer horizontalen Ebene eine normale Betriebsausrichtung hat, welche Anordnung die Füllung des Narkosemittelbehälters begrenzt, wenn der Narkosemittelbehälter bezüglich der horizontalen Ebene geneigt wird,
**dadurch gekennzeichnet, dass** ein Zwischenbehälter (5, 25, 35, 45, 55, 65, 75, 85), welcher aus einem gekrümmten rohrförmigen Teil gebildet ist, und sich im Wesentlichen in einer horizontalen Ebene in Bezug auf die normale Betriebsposition des Narkosemittelflüssigkeitsbehälters (1) befindet, in Verbindung mit dem Narkosemittelflüssigkeitsbehälter (1) vorgesehen ist, der Zwischenbehälter einen Durchflussweg zwischen dem Vorratsbehälter (2) und dem Inneren des Narkosemittelflüssigkeitsbehälters (1) bildet, der Zwischenbehälter (5, 25, 35, 45, 55, 65, 75, 85) eingerichtet ist, um eine gemeinsame Leitung für die Narkosemittelflüssigkeit und das von dem Narkosemittelflüssigkeitsbehälter entfernte Gas zu bilden, und der Zwischenbehälter (5, 25, 35, 45, 55, 65, 75, 85) eine Flüssigkeitsschwelle (NK) und eine Gasschwelle (KK) der Einfüllöffnung (4) und des Narkosemittelflüssigkeitsbehälters (1) definiert und die Flüssigkeitsschwelle (NK) und die Gasschwelle (KK) der Einfüllöffnung (4) und des Narkosemittelflüssigkeitsbehälters (1) derart kombiniert, dass es der Narkosemittelflüssigkeit nur erlaubt ist in den Narkosemittelflüssigkeitsbehälter (1) zu fließen, wenn die Flüssigkeitsschwelle (NK) unterhalb der Gasschwelle (KK) ist.

2. Anordnung nach Anspruch 1,
**dadurch gekennzeichnet, dass** das gekrümmte rohrformige Teil um wenigstens 90° in einer in Bezug auf die normale Betriebsposition des Narkosemittelflüssigkeitsbehälters (1) im Wesentlichen horizontalen Ebene gekrümmt ist.

3. Anordnung nach Anspruch 1,
**dadurch gekennzeichnet, dass** der Zwischenbehälter (5) ein im Wesentlichen U-förmiges rohrförmiges Teil ist.

4. Anordnung nach Anspruch 3,
**dadurch gekennzeichnet, dass** die Zweige des U-förmigen rohrförmigen Teils in einer in Bezug auf die normale Betriebsposition des Narkosemittelflüssigkeitsbehälters (1) im Wesentlichen horizontalen Position montiert sind.

5. Anordnung nach Anspruch 3,
**dadurch gekennzeichnet, dass** der höchste Punkt des unteren Teils des Zwischenbehälters (5) die Flüssigkeitsschwelle (NK) bildet, und der niedrigste Punkt des oberen Teils die Gasschwelle (KK) bildet.

6. Anordnung nach Anspruch 1,
**dadurch gekennzeichnet**, das das gekrümmte rahrförmige Teil als eine Spirale geformt ist.

7. Anordnung nach einem der vorhergehenden Ansprüche 1 bis 6,
**dadurch gekennzeichnet, dass** das rohrförmige Teil, welches den Zwischenbehälter (5, 25, 35, 75) bildet, aus einem Rohr gemacht ist.

8. Anordnung nach einem der vorhergehenden Ansprüche 1 bis 6,
**dadurch gekennzeichnet, dass** das rohrförmige Teil, welches den Zwischenbehälter (45, 55, 65, 85) bildet, aus zwei oder mehr Rohren gemacht ist.

9. Anordnung nach Anspruch 8,
**dadurch gekennzeichnet, dass** die Rohre in derselben Ebene montiert sind.

10. Anordnung nach Anspruch 8,
**dadurch gekennzeichnet, dass** die Rohre in verschiedenen Ebenen montiert sind.

11. Anordnung nach Anspruch 8, 9 oder 10,
**dadurch gekennzeichnet, dass** die Rohre nacheinander, parallel und/oder übereinander montiert sind.

12. Anordnung nach Anspruch 11,
**dadurch gekennzeichnet, dass** die Rohre sowohl auf derselben Ebene als auch auf verschiedenen Ebenen montiert sind.

13. Anordnung nach einem der vorhergehenden Ansprüche,
**dadurch gekennzeichnet, dass** der Zwischenbehälter (5, 25, 35, 45, 55, 65, 75, 85) zwischen der Einfüllöffnung (4) und dem inneren Raum des Narkosemittelflüssigkeitsbehälters (1) montiert ist.

14. Anordnung nach Anspruch 1,
**dadurch gekennzeichnet, dass** der Zwischenbehälter ein Teil umfasst, dessen Projektion in der vertikalen Richtung größer ist als der Durchmesser des Rohres.

## Revendications

1. Arrangement relatif à un récipient à liquide anesthésique d'un vaporisateur comportant des moyens pour canaliser le liquide anesthésique dans le récipient à liquide anesthésique (1) pour y être vaporisé et pour éliminer du récipient à liquide anesthésique (1) une volume de gaz équivalente à celle du liquide anesthésique y entrant, l'arrangement étant disposé à remplir de liquide anesthésique le récipient à liquide anesthésique (1) d'un vaporisateur au niveau désiré d'un récipient d'alimentation (2), ledit récipient à liquide anesthésique présentant une orientation d'opération normale par rapport au plan horizontal, ledit arrangement limitant le remplissage du récipient à liquide anesthésique lors que le récipient à liquide anesthésique est incliné par rapport au plan horizontal, **caractérisé en ce qu'**un récipient intermédiaire (5, 25, 35, 45, 55, 65, 75, 85), formé par une partie tubulaire courbée sensiblement au plan horizontal par rapport à la position d'opération du récipient à liquide anesthésique (1), est prévu en connexion avec le récipient à liquide anesthésique (1), ledit récipient intermédiaire constituant une voie de débit entre le récipient d'alimentation (2) et l'intérieur du récipient à liquide anesthésique (1), ledit récipient intermédiaire (5, 25, 35, 45, 55, 65, 75, 85) étant disposé à former un conduit commun pour le liquide anesthésique et le gaz éliminé du récipient à liquide anesthésique, et ledit récipient intermédiaire (5, 25, 35, 45, 55, 65, 75, 85) définissant un seuil de liquide (NK) et un seuil de gaz (KK) d'une entrée (4) de remplissage et le récipient à liquide anesthésique (1), et combinant le seuil de liquide (NK) et le seuil de gaz (KK) de l'entrée (4) de remplissage et le récipient à liquide anesthésique de manière qui permet au liquide anesthésique d'écouler dans le récipient à liquide anesthésique (1) seulement lors que le seuil de liquide (NK) est inférieur au seuil de gaz (KK).

2. Arrangement selon la revendication 1, **caractérisé en ce que** la partie tubulaire courbée est coudée au moins de 90 degrés au plan horizontal par rapport à la position d'opération normale du récipient à liquide anesthésique (1).

3. Arrangement selon la revendication 1, **caractérisé en ce que** le récipient intermédiaire (5) est une partie tubulaire sensiblement en U.

4. Arrangement selon la revendication 3, **caractérisé en ce que** les branchements de la partie tubulaire en U sont montées en position sensiblement horizontale par rapport à la position d'opération normale du récipient à liquide anesthésique (1).

5. Arrangement selon la revendication 3, **caractérisé en ce que** le point au plus haut de la partie inférieure du récipient intermédiaire (5) forme le seuil de liquide (NK) et le point au plus bas de la partie supérieure forme le seuil de gaz (KK).

6. Arrangement selon la revendication 1, **caractérisé en ce que** la partie tubulaire courbée est formée en spirale.

7. Arrangement selon l'une quelconque des revendications précédentes 1 à 6, **caractérisé en ce que** la partie tubulaire qui constitue le récipient intermédiaire (5, 25, 35, 75) est fabriquée d'un tube unique.

8. Arrangement selon l'une quelconque des revendications précédentes 1 à 6, **caractérisé en ce que** la partie tubulaire qui constitue le récipient intermédiaire (45, 55, 65, 85) est fabriquée de deux ou plusieurs tubes.

9. Arrangement selon la revendication 8, **caractérisé en ce que** les tubes sont montés au même niveau.

10. Arrangement selon la revendication 8, **caractérisé en ce que** les tubes sont montés aux niveaux différents.

11. Arrangement selon la revendication 8, 9, ou 10,
**caractérisé en ce que** les tubes sont montés en tandem, en parallèle ou en superposition.

12. Arrangement selon la revendication 11, **caractérisé en ce que** les tubes sont montés et au même niveau et aux niveaux différents.

13. Arrangement selon l'une quelconque des revendications précédentes, **caractérisé en ce que** le récipient intermédiaire (5, 25, 35, 45, 55, 65, 75, 85) est monté entre l'entrée (4) de remplissage et l'intérieur du récipient à liquide anesthésique (1).

14. Arrangement selon la revendication 1, **caractérisé en ce que** le récipient intermédiaire comporte une partie dont la projection dans le sens vertical est plus grande que celle du diamètre du tube.
